# EUROPEAN PATENT APPLICATION

(11) **EP 2 711 703 A1**
(43) Date of publication of application: **26.03.2014**
(21) Application number: 13447012.9
(22) Date of filing: 21.06.2013
(51) Int. Cl.: G01N 33/497, B60K 28/06, E01F 13/00

(54) **Autonomous apparatus and access control barrier for testing alcohol or drugs use for drivers**

(30) Priority: 19.09.2012 BE 201200614; 12.10.2012 BE 201200677; 12.10.2012 BE 201200675; 12.10.2012 BE 201200676
(71) Applicant: VAN DE SANDE, Dirk, 2222 Wiekevorst (BE)
(72) Inventor: VAN DE SANDE, Dirk, 2222 Wiekevorst (BE)

(57) **Abstract**

An autonomous alcohol or drugs test apparatus and parking access control with a barrier gate for vehicle drivers, and wherein the test apparatus, after testing, dispenses a coin or token to open the barrier gate or to request a new test and provided with identification and communication means.

## Description

### Field of the invention

The invention concerns an apparatus for testing the use of alcohol or drugs and a related parking access control for vehicle drivers. It comprises a test apparatus for measuring the amount of drugs or alcohol in the exhaled air or saliva of a vehicle driver, with coins or tokens dispensing means and an access barrier control on a vehicle parking. In particular, the test apparatus dispenses a coin or token that provides access to the road when the measured amount of alcohol is under the legal limit or no drugs detection, otherwise it dispenses a coin or token for a new test and prohibits road access for the driver.

### Background of the invention

The alcohol usage of a vehicle driver is legally limited and differs from country to country. It varies mainly from 0.2‰ to 0.8‰. This amount of alcohol is dependent of the kind and number of drinks,and the biological characteristics of the user, like his size and weight. Drugs means hard- or soft drugs, like narcotics, stimulants or hallucinogenic compounds that are prohibited. Opiates, benzodiazepines and barbiturates are examples of narcotics. Cocaine and XTC are categorised under stimulants. Cannabis, marihuana or LSD are hallucinogenic compounds. The use of these products during driving on the road increases the probability of an accident. Generally, drivers are controlled by police when already driving on the road, which cannot be considered as a preventive matter. Furthermore, excessive use of alcohol and drugs could harm the reputation of bars, restaurants, dancing locations or concert organisers. This means that a driver with a too high alcohol content or with a drug indication should not be allowed to drive on a public road.

However, alcohol and drugs test apparatuses are generally known in the state of the art.

GB2147099 describes an apparatus for measuring the alcohol content of a gas sample. It uses a coin-freed mechanism to start the analysis. The apparatus indicates with a lightning the measured alcohol content. This apparatus does not award or punish the user.

EP2196145 concerns a drinking level detecting system and a method by the intervention of a third person for determining if a driver would be able to leave a parking place.

DE10136576 describes a device to detect the use of drugs with a test strip and coupled to a laptop.

It is not known in the state-of-the-art to measure the alcohol or drug use and to control road access for a vehicle driver autonomously, without any human intervention with an apparatus that enables or prohibits to open a barrier gate on a vehicle parking.

### Summary of the invention

The invention concerns an autonomously awarding or punishing system for vehicle drivers, based on measured alcohol or drugs test levels with a test apparatus and a related parking access control. The test apparatus measures alcohol or drugs use in the exhausted air from the vehicle driver and based on the measured level, a coin or token will be dispensed that opens or prohibits the opening of a barrier gate of the parking.

Furthermore, according to an embodiment of the invention, the test apparatus is provided with biological identification means. The identification of the driver is stored in a memory module for recognition at the parking access barrier.

According to another embodiment of the invention, the test apparatus is provided with a communication module to check vehicle licence number plates in a database, to transmit a message to public services or to communicate with the barrier gate.

Another embodiment of this invention concerns an electronic coin or token, provided with a microchip and a memory module, a timer and/or communicating means.

### Detailed description of embodiments of the invention

The invention concerns a combination of an alcohol- or drugs test apparatus and a barrier gate at the parking access that enables an autonomous interaction. The test apparatus comprises a housing, wherein an alcohol or drugs measuring unit, visual or sound alerting means, a microprocessor, a memory module, two reservoirs with different coins or tokens, separated by a valve and coin or token inserting and dispensing means are provided. The test apparatus is further provided with paying means, such as a coin freed or card operating device to activate the apparatus; The wording 'autonomous' means that no human intervention is needed to determine the alcohol or drug intoxication of the vehicle driver. The wordings 'test apparatus' will be used for both the alcohol- or drugs test apparatus and 'coin' or 'coins' for coins or tokens that are used to operate a barrier gate or to re-use the test apparatus.

After inserting money in the test apparatus, the test apparatus enables a user to exhale or to blow in the measuring unit with a mouthpiece. The measuring unit comprises a sensor, an electrode or a test strip. In an embodiment of this invention a colour change, that signifies an intoxication, can be detected by a spectroscopic technique.

Based on the measured result, the user receives a visual or sound signal, respectively on a display or via a loudspeaker or a combination of both.

The test apparatus comprises two reservoirs with coins and dispensing means. Both reservoirs enable to dispense a coin via an outlet opening in the housing of the apparatus. The first reservoir holds coins to open a barrier at the parking exit. These coins are provided with a recognizable sign or colour, e.g. green. The second reservoir contains coins to reuse the test apparatus to perform a new test later. This colour can be e.g. red. An electro-mechanic or electro-magnetic valve is activated to dispense a coin from one of the reservoirs. After testing, the microprocessor activates the valve. When the measured amount is lower than the legal acceptable basis, which can be considered as acceptable, the valve receives a signal to dispense a green coin. This enables to open the barrier gate at the exit of the parking. When the alcohol level is too high, the valve opens the reservoir with the red coins and dispenses a red coin. This means that the driver cannot open the barrier gate and needs to request a new test later. In an embodiment according to this invention, the user receives a message that informs him over the time delay to request a new test.

The test apparatus further comprises, according to an embodiment of this invention, an identification module in order to control the identity of the vehicle driver. The identification module uses a biological characteristic, like an iris or a fingerprint recognition of the driver in order to check his identity at the barrier gate. According to another embodiment of the invention, a camera can be built in the test apparatus for recording and face recognition of the vehicle driver. The identification data of the vehicle driver are processed by the microprocessor and stored in a memory module.

Furthermore, according to another embodiment of this invention, the test apparatus is provided with a communication module. The communication module comprises a transmitter and receiver that establish a communication with the barrier gate to check the drivers' identity. According to another embodiment of this invention, the vehicle driver is requested to apply the vehicle licence number plate with an input device. The test apparatus enables to communicate with a,license number plate database to check the validity of the plate number and the identity of the driver. The vehicle licence number plate data are stored in the memory module. The communication module also provides a transmission between the test apparatus and a public service, such as the police in order to send a message.

The barrier gate is, according to an embodiment of this invention, also provided with biological identification means and a communication module, such as a transmitter and receiver. The barrier gate is further provided with a microprocessor that activates the identification means to check the drivers' identity.

According to another embodiment of the invention, electronic coins are provided with a microchip and a memory module to keep identification and measured data of the driver. Identification and test data, processed by the microprocessor are stored in the memory module and written to the memory of the electronic coin. The electronic coin is further provided with a timer and communicating means, such as a transmitter and receiver. The electronic coins are programmed to count the timing interval between a first test and future new test. Furthermore, when the driver does not use the coin within a predetermined time limit, the validity of the coin shall be exhausted.

When inserting the coin in the barrier gate to leave the parking, an identity check of the driver and the validity check of the coin are processed. When the identity data between the barrier gate and the stored data in the electronic coin or the transmitted data by the test apparatus correspond, the driver is allowed to leave the parking. In the latter case, the communication module of the test apparatus transmits the identity data to the barrier gate microprocessor. In the former case, the data on the microchip of the coin shall be read and compared by the microprocessor of the barrier gate.

## Claims

1. An autonomous alcohol or drugs test apparatus and parking access control with a barrier gate for vehicle drivers, whereby the.test apparatus comprises a housing with an alcohol or drugs measuring unit, visual or sound alerting means, a microprocessor, a memory module, two reservoirs with different coins or tokens, an electromechanical or electromagnetic valve and a coin or token inserting and dispensing mean **and characterised in that** after testing the alcohol or drugs use of a vehicle driver with the test apparatus, the valve is activated to dispense a coin or token to open the barrier gate from a first reservoir to provide road access or a coin or token to reuse the test apparatus for requesting a new test.

2. An autonomous alcohol or drugs test apparatus and parking access control according to claim 1 **and characterised in that** the test apparatus is provided with biological identification means to determine and to store the identity of the vehicle driver in a memory module.

3. An autonomous alcohol or drugs test apparatus and parking access control according to claims 1 or 2 **and characterised in that** the barrier gate is provided with a microprocessor that activates biological identification means.

4. An autonomous alcohol or drugs test apparatus and parking access control according to claims 2 or 3 **and characterised in that** the biological identification means comprise iris, fingerprint or face recognition means.

5. An autonomous alcohol or drugs test apparatus and parking access control according to claims 1 or 2 **and characterised in that** the test apparatus is provided with a communication module.

6. An'autonomous alcohol or drugs test apparatus and parking access control according to claim 1 or 3 **and characterised in that** the barrier gate is provided with a communication module.

7. An autonomous alcohol or drugs test apparatus and parking access control according to claims 5-6 **and characterised in that** the communication module establishes a communication between the test apparatus and the barrier gate.

8. An autonomous alcohol or drugs test apparatus and parking access control according to claim 5 **and characterised in that** the communication module of the test apparatus establish a communication between the test apparatus and a database with vehicle licence number plates.

9. An autonomous alcohol or drugs test apparatus and parking access control according to claim 5 **and characterised in that** the communication module provides a transmission between the test apparatus and a public service.

10. An autonomous alcohol or drugs test apparatus and parking access control according to claim 1 **and characterised in that** the coins or tokens in the reservoirs are electronic'coins or tokens, provided with a microchip and a memory module.

11. An autonomous alcohol or drugs test apparatus and parking access control according to claim 10 **and characterised in that** the identification of the driver, after using the test apparatus, is written and stored in the memory of the coin or token.

12. An autonomous alcohol or drugs test apparatus and parking access control according to claim 10 **and characterised in that** the electronic coins or tokens are provided with a communicated module.

13. An autonomous alcohol or drugs test apparatus and parking access control according to claim 10 **and characterised in that** the electronic coins or tokens are provided with a timer.

14. An autonomous alcohol or drugs test apparatus and parking access control according to claim 11 **and characterised in that** when inserting the coin or token in the barrier gate, the identity data of the driver, stored on the coin or token, is compared with the identification data determined at the barrier gate to open or to prohibit opening of the barrier gate.
